(19) Europäisches Patentamt
European Patent Office
Office européen des brevets

(11) Veröffentlichungsnummer: **0 143 362**
**B1**

(12) # EUROPÄISCHE PATENTSCHRIFT

(45) Veröffentlichungstag der Patentschrift: **02.08.89**

(51) Int. Cl.⁴: **A 61 K 6/08**, C 08 F 20/30

(21) Anmeldenummer: **84113226.9**

(22) Anmeldetag: **02.11.84**

(54) **Dentales Restorationsmaterial.**

(30) Priorität: **25.11.83 DE 3342601**

(43) Veröffentlichungstag der Anmeldung: **05.06.85 Patentblatt 85/23**

(45) Bekanntmachung des Hinweises auf die Patenterteilung: **02.08.89 Patentblatt 89/31**

(84) Benannte Vertragsstaaten: **AT BE CH FR GB IT LI LU NL SE**

(56) Entgegenhaltungen:
**DE-A-2 648 969**
**US-A-3 345 401**

**CHEMICAL ABSTRACTS, Band 98, Nr. 4, Januar 1983, Seite 339, Zusammenfassung Nr. 22237h, Columbus, Ohio, US; K.W.M. DAVY et al.: "Radioopaque denture base: a new acrylic copolymer"**

(73) Patentinhaber: **Blendax GmbH, Rheinallee 88, D-6500 Mainz (DE)**

(72) Erfinder: **Kuhlmann, Werner, Dr., Vogelsbergstrasse 11, D-6500 Mainz- Hechtsheim (DE)**

**EP 0 143 362 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein dentales Restorationsmaterial für die Füllung von Zahnkavitäten, das verbesserte Eigenschaften, insbesondere eine verbesserte Röntgenopazität, aufweist.

In der Dentalmedizin haben in den letzten Jahren besonders die aus Füllstoffen und polymerisierbaren Verbindungen bestehenden Füllungsmaterialien, die sogenannten "Composites", zunehmende Bedeutung erlangt, da diese Produkte vom Zahnarzt leicht und sicher zu handhaben sind, vom Patienten in der Regel gut und reizlos vertragen werden, ein ästhetisch ansprechendes Aussehen der Füllung gewährleisten und die Möglichkeit bieten, von den aus physiologischen Gründen immer wieder kritisierten Amalgam-Füllungsmaterialien abzurücken.

Unter den Anforderungen, die an die Eigenschaften solcher "Composites" gestellt werden, nehmen diejenigen nach Sichtbarkeit der Füllung unter Einfluß von Röntgenstrahlen, d. h. der Röntgenopazitat, und nach Polierbarkeit der Füllungsoberflächen einen hohen Stellenwert ein. Die Erfüllung dieser Forderungen mit den bislang bekannten "Composites" bereitet jedoch Probleme, da einerseits dann, wenn eine gut polierbare Füllstoffkombination, bestehend in der Regel aus größeren Anteilen feinteiliger Silikagele und geringen Anteilen an Bariumsilikatglas-Teilchen, eingesetzt wird, zwar eine gute Polierbarkeit, jedoch eine nicht ausreichende Röntgenopazität erreicht wird; andererseits aber dann, wenn der Anteil an röntgenopakem Füllstoff gesteigert wird, eine ausreichende Polierbarkeit der auf Basis dieser Zusammensetzung hergestellten ausgehärteten Füllung nicht mehr erreicht-werden kann.

Die vorliegende Erfindung löst dieses Problem in äußerst vorteilhafter Weise dadurch, daß in einem dentalen Restorationsmaterial auf Basis polymerisierbarer Verbindungen, Füllstoffen, Polymerisationskatalysatoren und/oder -beschleunigern und gegebenenfalls weiteren in solchen Mitteln üblichen Substanzen als polymerisierbare Verbindungen allein oder im Gemisch mit bekannten Substanzen mindestens eine oder mehrere Verbindungen der allgemeinen Formel

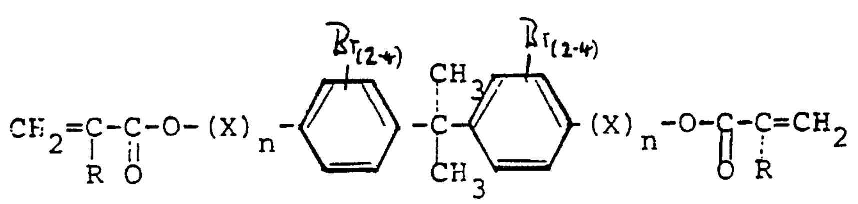

(I)

wobei R H o. Methyl,

X eine $CH_2$-$CH_2$-O-, $CH_2$-$CH_2$-$CH_2$-O- oder eine $CH_2$-CH(OH)-$CH_2$-O-Gruppe,

und n 0 bis 3 bedeuten,
eingesetzt werden.

Durch die Verwendung dieser Verbindungen wird auch beim Einsatz größerer Mengen eines sehr feinteiligen Füllstoffs, insbesondere Siliciumdioxid-Gelen mit mittleren Teilchendurchmessern zwischen 20 und 200 Nanometern, die zur Herstellung gut polierbarer Füllungswerkstoffe eingesetzt werden, nach dem Aushärten eine Füllung erhalten, die eine ausreichende Röntgenopazität aufweist.

Besonders vorteilhaft ist der Einsatz dieser speziellen Monomeren der allgemeinen Formel (I) bei der Herstellung von sogenannten lichthärtenden "Composites", d. h. Materialien, die in einer Phase vorliegen, in der Regel einen Photopolymerisationsinitiator enthalten und durch Lichteinfluß gehärtet werden können; jedoch ist selbstverständlich der Einsatz dieser Monomeren auch in sogenannten selbsthärtenden "Composites", die in zwei bis zur Anwendung voneinander getrennt gehaltenen Phasen vorliegen, möglich.

Gemäß einer bevorzugten Ausführungsform der Erfindung beträgt der Anteil der bromierten Monomeren nach der allgemeinen Formel (I) 30 bis 90 Gew.-%, vorzugsweise 55 bis 85, insbesondere etwa 60 bis 75 Gew.-% der Gesamtmenge der im dentalen Restorationsmaterial zum Einsatz gelangenden polymerisierbaren Substanzen.

Diese polymerisierbaren Substanzen sind in den erfindungsgemäßen dentalen Restorationsmaterialien in der Regel in einer Menge zwischen 15 und 50, vorzugsweise 20 bis etwa 40 Gew.-%, berechnet auf die Gesamtzusammensetzung des Restorationsmaterials, enthalten.

Der Rest des Mittels besteht demgemäß aus 50 bis 85, vorzugsweise 60 bis 80 Gew.-%, berechnet auf die Gesamtzusammensetzung, eines Füllstoffs oder eines Füllstoffgemisches mit einem mittleren Teilchendurchmesser von weniger als 30 Mikrometer und 0,05 bis 2,0 Gew.-% mindestens eines Polymerisationsinitiators und/oder Polymerisationsbeschleunigers sowie gegebenenfalls weiteren in solchen Mitteln üblichen Bestandteilen.

Gemäß einer bevorzugten Ausführungsform der Erfindung handelt es sich bei der bromierten polymerisierbaren Verbindung um eine solche der Formel

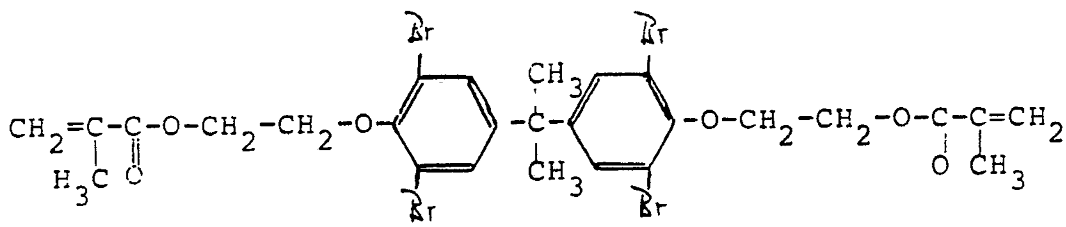

Die Herstellung dieser Verbindung kann durch Ethoxylierung des entsprechenden 2,2-Di-(3,5-Dibrom-4-hydroxyphenyl)propans und anschließende Veresterung des Ethoxylierungsproduktes mit Methacrylsäure geschehen.

In analoger Weise können auch die entsprechenden Hexa- und Octabrom-Verbindungen sowie Monoethoxy-Verbindungen der Struktur

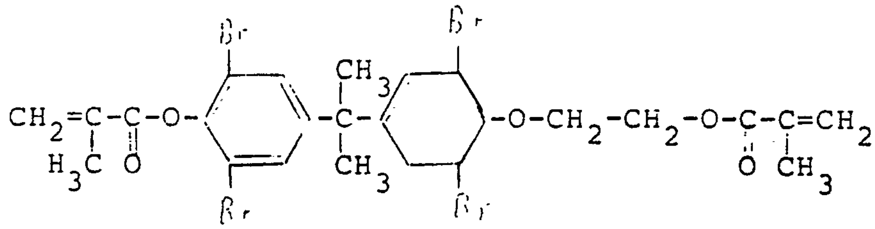

bzw. Triethoxy-Verbindungen oder, im Falle Umsetzung des bromierten Bis(hydroxyphenyl)propans mit Propylenoxid, auch die entsprechenden Propoxylate hergestellt werden. Verbindungen dieser Art und ihre Herstellung sind an sich bekannt, und beispielsweise in der DE-A-2 648 969, insbesondere in deren Beispiel 6, beschrieben, wo sie zur Herstellung von Copolymerisaten mit verringerter Entflammbarkeit benutzt werden.

Die Herstellung dieser Dimethacrylate kann auch nach den in der JP-A-5 795 941 (Chemical Abstracts, Vol. 98, No. 34 239y) sowie der JP-A-8 293 931 (Chemical Abstracts, Vol. 97, 183 393a) beschriebenen Herstellungsverfahren erfolgen.

Ein weiteres bevorzugt zum Einsatz gelangender Monomeres leitet sich von dem bekannten Reaktionsprodukt aus Bisphenol A und Glycidylmethacrylat ab und besitzt die Formel

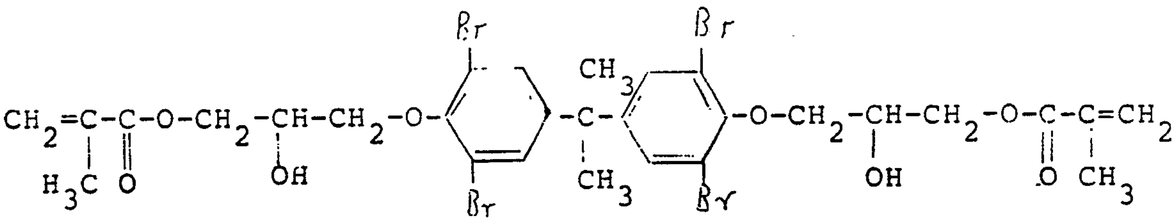

Solche Verbindungen sind aus der SU-A-747 850 bereits bekannt und werden dort zur Verbesserung der Hitzebeständigkeit zusammen mit anderen Monomeren, insbesondere Methylmethacrylat, copolymerisiert; diese Copolymeren weisen selbstverlöschende Eigenschaften auf.

Auch aus der DE-A-2 747 947 ist die Verwendung entsprechender Monomerer, nämlich des Di-(3-methacryloxy-2-hydroxypropyl)ethers und des Di-(2-methacryloxyethyl)ethers des Tetrabrom-Bisphenols A in photopolymerisierbaren Zusammensetzungen, die als Photoresists und Lötmasken verwendet werden sollen, bekannt.

Die DE-A-3 120 965 offenbart schließlich Copolymerisate, die zur Herstellung von Linsen mit einem hohen Brechungsindex, einer ausgezeichneten Durchsichtigkeit und Feuerbeständigkeit verwendet werden können, die bromierte Monomere enthalten, wie sie gemäß der allgemeinen Formel (I) in den erfindungsgemäßen dentalen Restorationsmaterialien zum Einsatz gelangen sollen.

In Anbetracht dieses Standes der Technik und der dort beschriebenen Anwendungsmöglichkeiten für diese bromierten Bisphenol-A-Methacrylate war es für den Fachmann höchst überraschend, daß sich diese auch als Bindemittel in dentalen Restorationsmaterialien einsetzen lassen und Füllungen mit optimalen Eigenschaften bezüglich der Röntgenopazität und der Polierbarkeit ergeben, wie sie mit den bisher für diesen Zweck verwandten Zusammensetzungen nicht erhaltbar sind.

Wie bereits ausgeführt, können die erfindungsgemäßen Restorationsmaterialien die Monomeren der allgemeinen Formel (I) als alleinige Bindemittel enthalten.

Es ist jedoch vorteilhaft, diese im Gemisch mit weiteren, an sich für diesen Zweck bekannten (Meth)Acrylsäureestern zu verwenden, insbesondere mit Alkandioldimethacrylaten wie 1,6-Hexandiolmethacrylat, Tri- und/oder Tetraethylenglycoldimethacrylat, 1,4-Butandioldimethacrylat, Trimethylolpropandi- und-trimethacrylat, Bis-(2-methacrylethyl)-phthalat -isophthalat oder -terephthalat, Reaktionsprodukten aus Diisocyanaten und einfachen Hydroxyalkylmethacrylaten, wie sie beispielsweise in der DE-A-2 312 559 beschrieben sind, Reaktionsprodukten aus unsubstituierten Bisphenolen, insbesondere Bisphenol A und Glycidylmethacrylat (Bis-GMA), -Addukten aus (Di)Isocyanaten und 2,2-Propanbis-3-(4-phenoxy)-1,2-hydroxypropan-1-methacrylat nach der US-A-3 629 187, den in der EP-A-44 352 beschriebenen Addukten aus Methacroylalkylethern, -alkoxybenzolen bzw. -alkoxycycloalkanen und Diisocyanaten, den aus der DE-B-2 357 324 und der DE-A-2 419 887 sowie der US-A-3 425 988 bekannten (Meth)Acrylsäureestern mit Carbaminsäuregruppierungen im Molekül sowie den in der GB-A-2 079 297 beschriebenen Reaktionsprodukten aus Diisocyanaten und Hydroxyalkyldiacrylaten und -methacrylaten sowie sonstigen für diesen Zweck bereits vorgeschlagenen polymerisierbaren Verbindungen.

Wie bereits ausgeführt, beträgt der bevorzugte Gehalt an bromierten Methacrylsäureverbindungen der allgemeinen Formel (I) 30 bis 90, vorzugsweise 55 bis 85, insbesondere 60 bis 75 Gew.-% des Anteils der gesamten polymerisierbaren Verbindungen.

In den erfindungsgemäßen Restorationsmaterialien machen die Füllstoffe in der Regel mehr als die Hälfte der Gesamtzusammensetzung aus.

Bei den eingesetzten Füllungsmaterialien kann es sich dabei um röntgendurchlässige oder röntgenundurchlässige Stoffe handeln. In jedem Fall kann der Anteil des in der Regel einen höheren mittleren Teilchendurchmesser aufweisenden und dadurch die Polierfähigkeit der Füllung verringernden, röntgenundurchlässigen Materials aufgrund der Eigenschaften der bromierten Monomeren soweit herabgesetzt werden, daß eine ausreichende Polierbarkeit der Füllung bei gleichzeitiger Beibehaltung der Röntgenopazität erreicht wird. Es ist sogar möglich, auf röntgenundurchlässige Füllstoffe gegebenenfalls ganz zu verzichten.

Beispiele für geeignete Füllstoffe sind insbesondere die verschiedenen Siliciumdioxid-Modifikationen wie kolloidale(d.h. gefällte oder pyrogen hergestellte) Silikagele, Glas (pulverisiertes Glas), Borsilikatglas undandere Gläser wie Quarzit, Cristobalit u.ä., Glaskeramik-Füllstoffe, Bariumaluminiumsilikat, Lithiumaluminiumsilikat, und Gläser, die Seltenerd-Elemente wie Lanthan oder Zirkonium enthalten.

Geeignete Füllstoffe sind beispielsweise in den US-A-3 801 344, 3 808 170 und 3 975 203 sowie der DE-A-2 347 591 beschrieben.

Zur Erhöhung der Affinität zwischen Bindemittel und Füllstoffen können letztere in an sich bekannter Weise silanisiert sein.

Die Teilchengrößen der eingesetzten Füllstoffe bewegen sich in der Regel zwischen 0,01 und 30 Mikrometern.

Einen bevorzugten Füllstoff stellt eine Kombination aus einem Bariumaluminiumsilikatglas mit einer mittleren Teilchengröße zwischen 3 und 10 Mikrometern und einem feinteiligen kolloidalen Siliciumdioxid mit einem mittleren Teilchendurchmesser zwischen 30 und 100 Nanometern dar, wobei das feinteilige Siliciumdioxid vorzugsweise im Überschuß, beispielsweise im Verhältnis 2 : 1, zum Bariumsilikatglas vorliegt.

Geeignete Füllstoffe sind auch die in der EP-A-60 911 beschriebenen, hochsilanisierten Siliciumdioxide und die aus der US-A-4 388 069 bekannten Gemische aus amorphen und kristallinen Füllstoffen.

"Composites" können in zwei Modifikationen vorliegen, entweder als zweiphasige Präparate, von den die eine Phase einen Polymerisationsinitiator, beispielsweise ein Peroxid, und die andere Phase einen Beschleuniger für dieses Peroxid, beispielsweise ein Amin, enthält, wobei das Zusammenbringen beider Phasen unmittelbar vor der Zahnfüllung erfolgt und die Polymerisation in der aufgebohrten, vorzugsweise mit einem Unterfüllungsmaterial versehenen, zu füllenden Kavität eintritt.

Die andere Modifikation eines "Composites", die erfindungsgemäß bevorzugt wird, sind einphasige Präparate, die unter Einwirkung von Licht polymerisieren und üblicherweise einen Photopolymerisationsinitiator und ggf. auch einen Beschleuniger hierfür enthalten.

Derartige Photopolymerisationsinitiatoren sind bekannt; vorzugsweise handelt es sich dabei um Carbonylverbindungen, insbesondere Benzil und Benzilderivate wie 4,4-Oxydibenzil oder andere Dicarbonylverbindungen, z. B. Diacetyl, 2,3-Pentandion, oder Metallcarbonyle, Chinone, insbesondere Campherochinon, und deren Derivate.

Charakteristische Photopolymerisationsinitiatoren für dentale Füllungsmaterialien sind beispielsweise in der DE-A-2 126 419 beschrieben.

Der Anteil an Photopolymerisationsinitiatoren beträgt vorzugsweise etwa 0.01 bis etwa 1,0 Gew.-% der Gesamtzusammensetzung. Diese lichthärtbaren dentalen Füllungsmaterialien enthalten vorzugsweise auch noch sogenannte Polymerisationsbeschleuniger.

Als solche sind insbesondere die verschiedenen Amine wie p-Toluidin, Dimethyl-p-toluidin, Dimethyl- und Diethylaminoethylmethacrylat, Trialkylamine, Polyamine, Dialkylbarbitursäuren und Sulfimide, vorzugsweise in einer Menge von 0,01 bis 2,5 Gew.-% der Gesamtzusammensetzung, geeignet.

Soll das erfindungsgemäße dentale Restorationsmaterial nicht lichthärtbar sein und in zwei bis zur Anwendung voneinander getrennt gehaltenen Phasen vorliegen, so enthält eine dieser Mischungen in der Regel einen Polymerisationsinitiator. Dies sind zumeist Peroxide, die bei der Auslösung der Polymerisation unter Radikalbildung zerfallen. Geeignete Peroxide sind beispielsweise Arylperoxide wie Benzoylperoxid, Cumolhydroperoxid, Harnstoffperoxid, tert.-Butylhydroperoxid oder -perbenzoat und Silylperoxide, in Mengen von vorzugsweise 0,05 bis 5, insbesondere 0,5 bis 2,5 Gew.-% der Gesamtzusammensetzung.

Enthält die eine Phase des zweiphasigen Mittels einen Polymerisationsinitiator, so wird der anderen Phase zweckmäßigerweise ein Beschleuniger des oben beschriebenen Typs, vorzugsweise ein Amin, zugesetzt.

Zur Einstellung eines möglichst naturgetreuen Eindrucks der gefüllten Zahnflächen enthalten Composite-Materialien zuweilen Farbstoffe in geringen Mengen.

Weiterhin ist der Einsatz geringer Mengen an UV-Stabilisatoren möglich. Geeignete Stabilisatoren sind z. B. Hydrochinon, p-Benzochinon, p-Butylhydroxytoluol, Propylgallat, u.ä.

Die folgenden Beispiele dienen der Erläuterung der Erfindung:

**Beispiel 1**

| | |
|---|---|
| Silanisiertes pyrogen hergestelltes Silicagel des Typs Ärosil® (mittl. Teilchendurchmesser 40 - 50 nm) | 43,7 (Gewichtsteile) |
| Silanisiertes Bariumsilikat-Glas (mittl. Teilchendurchmesser 3 - 10 µm) | 25,6 |
| 1,6-Hexandioldimethacrylat | 10,7 |
| Isopropylidenbis-[2-(3,5-dibrom-4-phenoxy)ethylmethacrylat] | 19,5 |
| Dimethylaminoethylmethacrylat | 0,3 |
| Campherochinon | 0,1 |
| Ethylbenzoin | 0,1 |
| UV-Stabilisatoren, Farbstoffe, optische Aufheller | q.s. |

Beim Aushärten dieses lichthärtenden Composites wurde ein hochglanzpolierbares, röntgenopakes Material erhalten.

Der Ersatz des Isopropylidenbis-[2-(3,5-dibrom-4-phenoxy)ethylmethacrylats] durch die unbromierte, in Composites üblicherweise eingesetzte analoge Verbindung ergab eine zwar polierbare, jedoch nicht röntgenopake Füllung.

**Beispiel 2**

| | |
|---|---|
| Silanisiertes pyrogen hergestelltes Silicagel des Typs Ärosil® (mittl. Teilchendurchmesser 60 nm) | 55,4 (Gewichtsteile) |
| 1,6-Hexandioldimethacrylat-Prepolymerisat | 6,0 |
| Triethylenglycoldimethacrylat | 8,0 |
| Isopropylidenbis-[2-(3,5-dibrom-4-phenoxy) ethylmeyhacrylat] | 30,0 |
| Diethylaminoethylmethacrylat | 0,4 |
| Campherochinon | 0,1 |
| Ethylbenzoin | 0,1 |
| UV-Stabilisator, Pigmente, optischer Aufheller | q.s. |

Beim Aushärten dieses Composites durch Belichten wird eine hochglanzpolierbare, röntgenopake Füllung erhalten.

**Beispiel 3**

| | |
|---|---|
| Silanisiertes kolloidales Silicagel (mittl. Teilchendurchmesser 50 - 100 nm) | 42,8 (Gewichtsteile) |
| Silanisiertes Quarzpulver (mittl. Teilchendurchmesser 3 - 8 µm) | 12,5 |
| Triethylenglycoldimethacrylat | 15,5 |
| Isopropylidenbis-[2-hydroxy-3-(3,5-dibrom-4-phenoxy)propylmethacrylat] | 28,5 |
| Campherochinon | 0,15 |
| Dimethylaminoethylmethacrylat | 0,4 |
| Ethylbenzoin | 0,15 |
| UV-Absorber, optische Aufheller | q.s. |

Die Aushärtung dieser Zusammensetzung ergab ein hochglanzpolierbares, röntgenopakes Füllungsmaterial.

Der Ersatz des bromierten Monomeren durch eine gleiche Menge des Grundkörpers Isopropylidenbis-[2-hydroxy-3-(4-phenoxy)propylmethacrylat] führte zu einem röntgendurchlässigen Füllungsmaterial.

**Beispiel 4**

| | Komponente A | Komponente B |
|---|---|---|
| Silanisiertes, pyrogen hergestelltes Silicagel (mittl. Teilchendurchmesser 30 - 60 nm) | 45,0 | 45,0 (Gewichtsteile) |
| Silanisiertes Bariumsilikat-Glas (mittl. Teilchendurchmesser 5 - 10 µm) | 25,0 | 25,0 |
| Triethylenglycoldimethacrylat | 10,4 | 10,4 |
| Isopropylidenbis-[2-(3,5-dibrom-4-phenoxy)ethylmethacrylat] | 19,0 | 19,0 |
| N,N-Dihydroxyethyl-p-toluidin | 0,5 | |
| Benzoylperoxid | | 0,4 |
| UV-Stabilisator, Farbstoffe | q.s. | q.s. |

Nach dem Zusammenbringen beider Komponenten und erfolgter Aushärtung wurde ein hochglanzpolierbares, röntgenundurchlässiges Produkt erhalten.

**Patentansprüche**

1. Dentales Restorationsmaterial, enthaltend polymerisierbare Verbindungen, Füllstoffe, Polymerisationskatalysatoren und/oder Polymerisationsbeschleuniger und gegebenenfalls weitere in solchen Mitteln übliche Substanzen, dadurch gekennzeichnet, daß die polymerisierbare Verbindung ganz oder teilweise aus einer oder mehreren Verbindungen der allgemeinen Formel

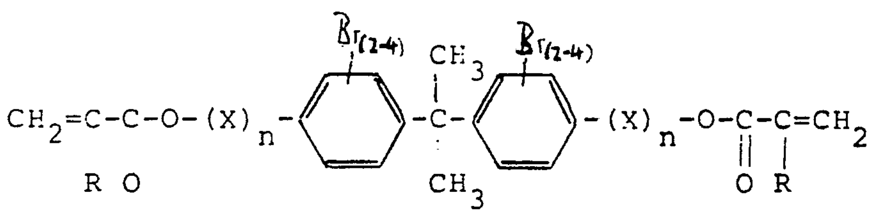

(I)

wobei
R H o. Methyl,

X eine $CH_2$-$CH_2$-O-, eine $CH_2$-$CH_2$-$CH_2$-O- oder eine $CH_2$-CH-$CH_2$-O-Gruppe,
OH

und n 0 bis 3 bedeuten,
besteht.

2. Dentales Restorationsmaterial nach Anspruch 1, dadurch gekennzeichnet, daß die polymerisierbaren Verbindungen zu 30 bis 90 Gew.-%, vorzugsweise 55 bis 85 Gew.-% (berechnet auf den Gesamtgehalt polymerisierbarer Verbindungen), aus einer oder mehreren Verbindungen der allgemeinen Formel (I) bestehen.

3. Dentales Restorationsmaterial nach Anspruch 1 und/oder 2, gekennzeichnet durch einen Gehalt an einer Verbindung der Formel

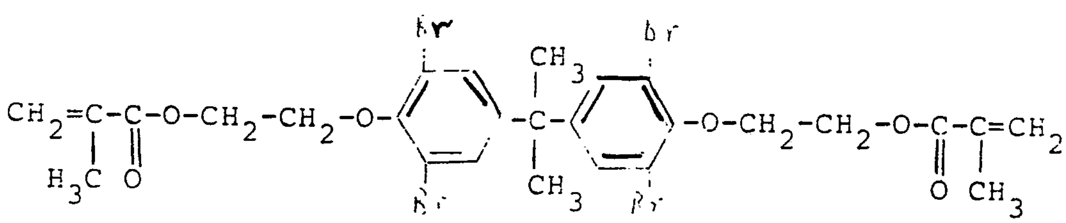

4. Dentales Restorationsmaterial nach Anspruch 1 und/oder 2, gekennzeichnet durch einen Gehalt an einer Verbindung der Formel

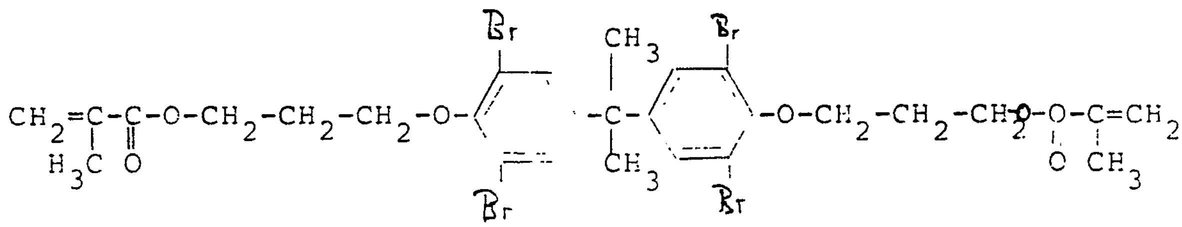

5. Dentales Restorationsmaterial nach Anspruch 1 und/oder 2, gekennzeichnet durch einen Gehalt an einer Verbindung der Formel

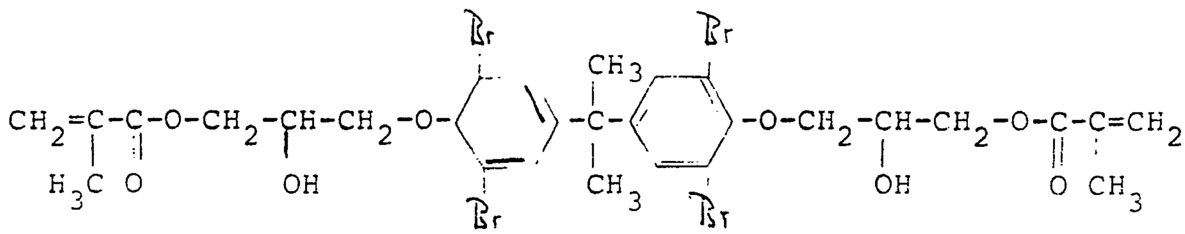

6. Dentales Restorationsmaterial nach Anspruch 1 und/oder 2, enthaltend

15 bis 50, vorzugsweise 20 bis 40 Gew.-%, berechnet auf die Gesamtzusammensetzung, polymerisierbare Verbindungen, enthaltend mindestens eine Verbindung der allgemeinen Formel (I);

50 bis 85, vorzugsweise etwa 60 bis etwa 80 berechnet auf die Gesamtzusammensetzung, mindestens eines Füllstoffs mit einem mittleren Teilchendurchmesser von weniger als 30 µm;

0,05 bis 2,5 Gew.-% mindestens eines Polymerisationsinitiators und/oder Polymerisationsbeschleunigers; gegebenenfalls weitere in solchen Mitteln übliche Bestandteile.

7. Dentales Restorationsmaterial nach einem oder mehreren der vorhergehenden Ansprüche, dadurch gekennzeichnet, daß es in Form einer einphasigen, lichthärtbaren Zusammensetzung vorliegt.

## Claims

1. A dental restoration material containing polymerisable compounds, fillers, polymerisation catalysts and/or polymerisation accelerators and optionally other substances customary in such compositions, characterised in that the polymerisable compound consists wholly or partly of one or more compounds of the general formula

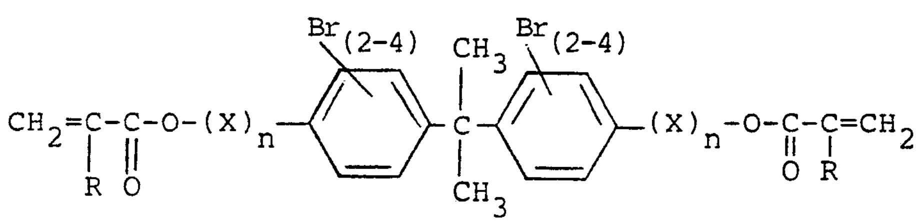

(I)

in which

R represents hydrogen or methyl, >t

X represents a $CH_2$-$CH_2$-O-, $CH_2$-$CH_2$-$CH_2$-O- or $CH_2$-CH-$CH_2$-O- group, and
OH

n is from 0 to 3.

2. A dental restoration material according to claim 1, characterised in that from 30 to 90 % by weight, preferably from 55 to 85 % by weight, of the polymerisable compounds (based on the total content of polymerisable compounds) consists of one or more compounds of the general formula (I).

3. A dental restoration material according to claim 1 and/or 2, characterised by a content of a compound of the formula

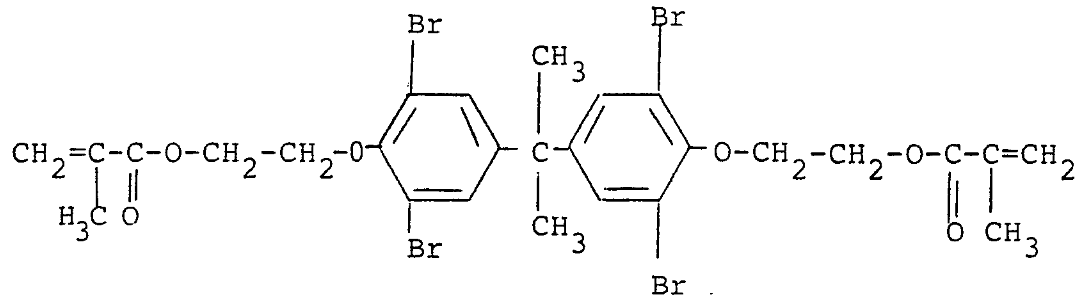

4. A dental restoration material according to claim 1 and/or 2, characterised by a content of a compound of the formula

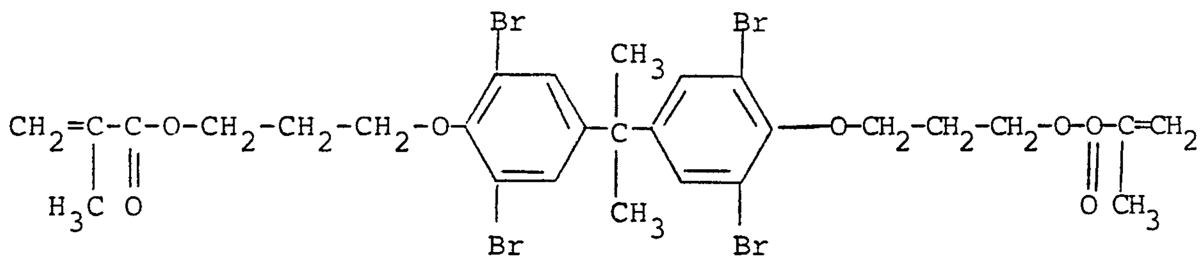

5. A dental restoration material according to claim 1 and/or 2, characterised by a content of a compound of the formula

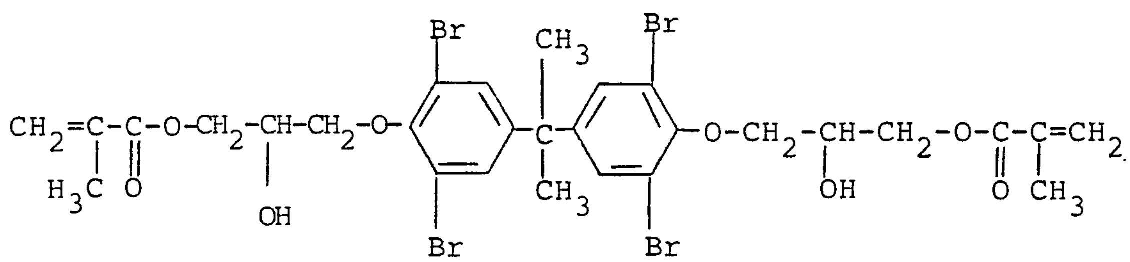

6. A dental restoration material according to claim 1 and/or 2 containing

from 15 to 50 % by weight, preferably from 20 to 40 % by weight, based on the total composition, of polymerisable compounds, containing at least one compound of the general formula (I);

from 50 to 85 % by weight, preferably from approximately 60 to approximately 80 % by weight, based on the total composition, of at least one filler having an average particle diameter of less than 30 μm;

from 0.05 to 2.5 % by weight of at least one polymerisation initiator and/or polymerisation accelerator; and optionally other components customary in such compositions.

7. A dental restoration material according to one or more of the preceding claims, characterised in that it is in the form of a single-phase, light-curable composition.

## Revendications

1. Matériau de restauration dentaire, contenant des composes polymérisables, des charges, des catalyseurs de polymérisation et/ou des accélérateurs de polymérisation et éventuellement d'autres substances courantes dans de tels produits, caractérisé en ce que le composé polymérisable est constitué totalement ou partiellement d'un ou plusieurs composés de la formule générale

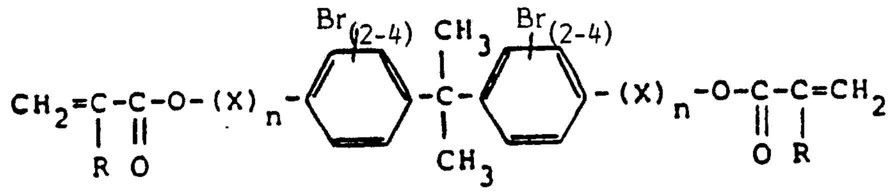 (I)

dans laquelle
R représente H ou du méthyle,

X représente $CH_2$-$CH_2$-O-, $CH_2$-$CH_2$-$CH_2$-O- ou$CH_2$-CH-$CH_2$-O- et n a une valeur de 0 à 3.
|
OH

2. Matériau de restauration dentaire suivant la revendication 1, caractérisé en ce que les composés polymérisables sont constitués pour 30 à 90 % en poids, de préférence 55 à 85 % en poids (calculés sur la teneur totale des composés polymérisables), d'un ou de plusieurs composés de la formule générale 1.

3. Matériau de restauration dentaire suivant la revendication 1 et/ou 2, caractérisé par une teneur en un composé repondant à la formule

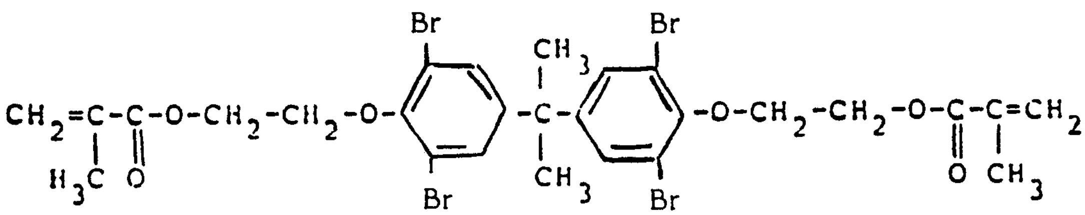

4. Matériau de restauration dentaire suivant la revendication 1 et/ou 2, caractérisé par une teneur en un composé de la formule

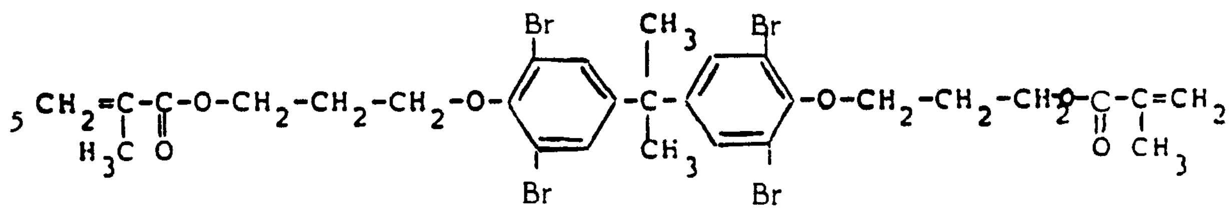

5. Matériau de restauration dentaire suivant la revendication 1 et/ou 2, caractérisé par une teneur en un composé de la formule

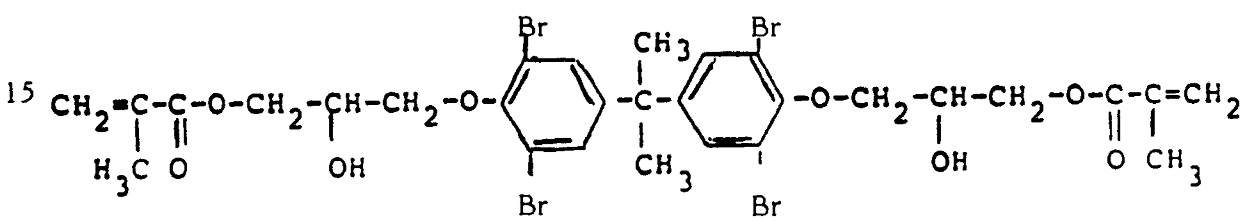

6. Matériau de restauration dentaire suivant la revendication 1 et/ou 2, contenant 15 à 50 % en poids, de préférence 20 à 40 % en poids, calculés sur la composition globale, de composés polymérisables contenant au moins un composé de la formule générale I,

50 à 85 % en poids, de préférence environ 60 à environ 80 % en poids, calculés sur la composition globale, d'au moins une charge ayant un diamètre moyen des particules inférieur à 30 µm,

0,05 à 2,5 % en poids d'au moins un initiateur de polymérisation et/ou d'un accélérateur de polymérisation, éventuellement d'autres éléments courants dans de tels produits.

7. Matériau de restauration dentaire suivant une ou plusieurs des revendications précédentes, caractérisé en ce qu'il se présente sous la forme d'une composition à une phase, durcissable à la lumière.